# EUROPEAN PATENT SPECIFICATION

(11) **EP 0 431 833 B1**
(45) Date of publication and mention of the grant of the patent: **28.02.1996**
(21) Application number: 90312966.6
(22) Date of filing: 29.11.1990
(51) Int. Cl.: A01H 1/02, A23D 9/00

(54) **Low linolenic acid oil producing flax cultivars**
Öl mit niedrigem Gehalt von Linolensäure produzierender Flachskultivars
Cultivars de lin produisant huile avec teneur bas d'acide linolenique

(30) Priority: 29.11.1989 US 443668
(43) Date of publication of application: 12.06.1991
(73) Proprietor: COMMONWEALTH SCIENTIFIC AND INDUSTRIAL RESEARCH ORGANISATION, Campbell, Australian Capital Territory 2601 (AU)
(72) Inventor: Green, Allan Graham, Australian Capitol Territory 2602 (AU)
(74) Representative: Sheard, Andrew Gregory

(56) References cited:
- EP-A- 0 092 076
- EP-A- 0 323 753
- EP-A- 0 326 198
- CROP SCIENCE, vol. 3, 1963, pages 171-172; V.E. Comstock et al.: "Association among seed an agronomic characteristics in isogenic lines of flax"
- PLANT BREEDING, vol. 98, 1987, pages 89-96, Paul Parey Scientific Publishers, Berlin, DE; N.N. ROY et al.: "Prospects for the development of rapeseed (B. napus L.) with improved linolenic acid content"
- ZEITSCHRIFT FÜR PFLANZENZÜCHTUNGm vik, 75, no. 2, 1975, Verlag Paul Parey, Berlin, DE; A. NITSCH: "Genetical and physiological investigations on mutants for polyenoic fatty acids in rapeseed, brassica napus L. I.Selection and description of new mutants"
- R.W. ALLARD: "Principles of plant breeding", 1960, John Wiley & Sons, New York, Chapter 35, pages 444-454: "Mutation breeding"

## Description

The present invention relates generally to flax and, more particularly, to flax cultivars, having low-linolenic acid content produced by genetic manipulation through induced mutations and subsequent breeding.

N N Roy *et al, Plant Breeding* **98**: 89-96 (1987) refers to the development of rapeseed with improved linoleic and linolenic acid content using cross-breeding techniques.

Linum usitatissimum or flax has traditionally been utilized as a source of industrial quality oil, linseed oil, for use in the production of paints, varnishes, inks and linoleum. The high level of linolenic acid in the oil, usually greater than 45%, imparts the rapid drying property necessary in such products. Due to the increased use of synthetic bases in these products, the demand for linseed oil has declined markedly in recent years. From 1971 to 1986, world production of linseed oil declined by 24% from 1,011,000 metric tons to 764,000 metric tons.

The market for edible vegetable oil is, however, a large and expanding one, but linseed oil cannot be utilized in edible products in its natural form because its high linolenic acid content causes rancidity due to autoxidation. Linolenic acid is an unsaturated fatty acid of the formula cis-9-, cis-12-, cis-15- octadecatrienoic acid which can be represented as 18:3 (carbon atoms:double bonds).

In order to convert linseed oil into a premium edible oil, linolenic acid would have to be completely eliminated, although a reduction to around 5% of total fatty acids would make the oil useful as a lower grade cooking and salad oil. Extensive surveys of L. usitatissimum germplasm collections reveal that variety mean values for linolenic acid content vary only in the range of 45% to 65%, with some of the variability within the variety arising from growth temperature.This observation leads to the conclusion that hybridization and selection within the available germplasm would be unlikely to achieve the required reduction in linolenic acid content.

There is, therefore, a need to develop low-linolenic acid content cultivars employing means other than hybridization and selection from within presently available germplasm.

Downey and Dorrell, Proc. Flax Inst. U.S.A. (1971) 41:1-3, report efforts by flax breeders to identify low-linolenic acid genotypes that might find a place in the edible oil markets. Zimmerman and Klosterman, Proc. Nth. Dakota Acad. Sci. (1959) 13:71-75, report extensive surveys of L. usitatissimum germplasm collection's revealing the variety mean values of linolenic acid content. Green and Marshall, Aust. J. Agric. Res. (1981) 32:599-607, describe the improbability of obtaining low-linolenic acid content cultivars by hybridization and selection within the available germplasm reported by Zimmerman and Klosterman.

Mutation breeding has been successful in reducing the content of polyenoic fatty acids in oilseeds. Rakow, Z. Pflanzenzuchtg (1973) 69:62-82 reports the reduction of linolenic acid content in rapeseed by inducing mutations with ethyl methanesulfonate. Robbelen and Nitsch, Z. Pflanzenzuchtg (1975) 75:93-105 describe the further reduction of linolenic acid content in Rakow's M57 rapeseed mutant by remutation. Hammond, et al., J. Am. Oil Chem. Soc. (1972) 49:33-35 describe similar reductions in linolenic acid content in soybean following X-ray mutagenesis. Srinivasachar and Malik, Current Sci. (1971) 40:298-99 report increased variability in the proportion of unsaturated fatty acids in linseed following treatment with gamma rays. Vereshchagin, Biokhimiya (1973) 38:573-82 reports similar results with diethylsulfate treatment of linseed.

A number of studies report that yellow-seeded flax generally contains higher levels of linolenic acid than brown-seeded varieties. Among these references are Culbertson and Kommendahl, Agronomy J. (1956) 48:25-28, Culbertson et al., Agronony J. (1960) 52:210-212, Comstock et al., Crop Sci. (1963) 3:171-172 and Comstock et al., Crop Sci. (1969) 9:513-514. The latter report also characterises the heredity of the yellow colour trait.

The present invention thus seeks to provide flax cultivars that have low linolenic acid content resulting in the production of seeds containing edible linseed oil. These cultivars may produce an oil superior to rapeseed oil and soybean oil in terms of higher linoleic acid content but lower linolenic acid content. In the art the general name, 'Linola', will usually designate yellow-seeded low linolenic acid containing flax plants.

Thus according to a first aspect of the present invention there is provided a flax plant (such as the genus Linum, e.g. species Linum usitatissimum) having a genetic mutation induced by treatment with a chemical or exposure to ionising radiation or by any other non-natural process, which affects the biosynthesis pathway for converting linoleic acid to linolenic acid so that the plant produces a seed oil having a linolenic acid content of less than about 45% of the total fatty acid content.

The genetic mutation is induced by any suitable non-natural process, such as chemically, for example by treatment with a chemical, or by exposure to ionising radiation, such as alpha or beta particles or gamma rays. Chemically induced mutation is preferred, and such a chemical is suitably a mutagen, for example a sulphonate. Preferred mutagens include C₁₋₄ (e.g. C₂) alkyl sulphonates and their derivatives, such as diethyl sulphonate and/or ethyl methane sulphonate.

Thus the present invention contemplates propagating material (such as a plant or seed) that has been chemically treated by a chemical (or exposed to ionising radiation) to affect the biosynthetic pathway for converting linoleic acid to linolenic acid.

Preferably the biosynthetic pathway is affected in the sense that the conversion of linoleic acid to linolenic acid is decreased or inhibited (or even prevented). Thus the effect of the, e.g. chemical, in this case will generally be to decrease the amount of linolenic acid present. The biosynthetic pathway is affected to the extent that the seed oil has less than about 45% of linolenic acid based on the total fatty acid content.

The preferred flax cultivars (or plants: the terms are used interchangeably) may thus be derived from flax seed having induced genetic mutations, such as at two unlinked loci exhibiting additive gene action. The induced mutations may specifically affect the linoleic acid desaturation step in fatty acid biosynthesis. This may cause the contents of linoleic acid to exceed that of linolenic acid. It is preferred that two mutations are induced in the flax plants of the present invention.
The linoleic acid content of the seed oil of the plants of the invention is preferably in excess of 10% of the total fatty acid content. Preferably the seed oil has a linolenic acid content of from 0% to 30% of the total fatty acid content. Thus suitably the seed oil of the flax plants has a linolenic acid content of less than 30% (and more preferably less than 15%) of the total fatty acid content. Advantageously linoleic acid content in the seed oil is greater than the linolenic acid content.

By inducing two mutations, one can obtain flax plants wherein the linolenic acid content in the seed oil can be from 5 to 12%, preferably from 0% to 12% (or from 0% to 5%) of the total fatty acid content. Thus a preferred flax plant is capable of producing a seed oil which has a linolenic acid content of less than 12% of total fatty acid content and a linoleic acid content of greater than 22% of total fatty acid content. These plants are preferably bred to provide a yellow seed coat.

A second aspect of the present invention relates to a plant which is the progeny (such as by cross-breeding) of a flax plant of the first aspect having the same genetic mutation as, and producing the seed oil of, a plant of the first aspect.

The present invention encompasses flax cultivars having low linolenic acid content. These 'Linola' cultivars may be developed by the inbreeding of crosses between low linolenic acid content flax seeds and other genotypes as required to achieve superior agronomic performance, seed distinctiveness or the like.

The genotypes of the stock cultivars for breeding are the result of selection within the genetically diverse breeding populations generated by intercrossing the aforementioned genotypes and self-pollinating their progeny through several generations until completely inbred. The resulting stock cultivars are true-breeding and uniform in appearance.

The cultivars included in the present invention may thus be produced by a combination of induced mutation, selection, cross-breeding and in-breeding of plants and progeny through several generations. This process may result in obtaining flax plants which produce seeds containing oil having a low linolenic acid content, such as from 0% to 30%, preferably from 0% to 15%, and more particularly from 0% to 5%, of the total fatty acid content.

The cultivars include both true breeding stock cultivars, hybrids which do not breed true and synthetic mixtures of low linolenic acid producing flax plants.

The reduced linolenic acid producing seeds may then be selected from plants grown from mutagenically treated flax seed. Linolenic acid content is determined either quantitatively by gas chromatography, or semi-quantitively by the TBA (thiobarbituric acid) test. The tests may be performed, either destructively or non-destructively, on the seeds and are known to those skilled in the art.

Cross-breeding and in-breeding of the plants of the present invention may be achieved by standard plant breeding techniques well known to those skilled in the art. Since the linoleic acid desaturation step in fatty acid biosynthesis is apparently regulated in flax by two unlinked loci exhibiting additive gene action, cross-breeding and subsequent in-breeding of reduced linolenic acid mutants may facilitate the selection of homozygous, true-breeding cultivars which are sexually propagated by self-pollination.

The present invention also allows for the breeding of desirable agronomic characteristics and distinctiveness into the cultivars. Cross-breeding of low-linolenic acid plants of the first aspect with disease resistant flax varieties, or regionally adapted varieties, or the like may be accomplished and are within the scope of the present invention. Also, distinctiveness of cultivars may be desired. Such distinctions may be achieved for seed and/or plant through colour, configuration or the like. For example, yellow seed colour is preferred and achieved through cross-breeding with yellow seed varieties of flax. Heterozygous plants resulting from crosses between yellow-seeded wild-type flax and low-linolenic acid plants of the invention may demonstrate dominance of both the yellow seed and low-linolenic acid phenotypes.

Oil may be derived from flax seed either directly or from plant parts by methods well known to those skilled in the art. (Lathlean, NSW Agricultural Gazette (April 1979, pages 2-5.)

### 'Linola' cultivars

Yellow seeded, low linolenic oil producing 'Linola' cultivars can be developed from crosses between the genotype 'Zero', imparting low-linolenic acid content, CPI 90432, (Ex. Red River Commodities, Fargo, ND), CPI 84495 (Ex. University of Birmingham, U.K.) or other genotypes imparting yellow seed colour, and any other genotypes as required to achieve superior agronomic performance, especially high seed yield (greater than 0.9 ton per hectare), adaptation to a wide range of environments and resistance to major diseases such as flax rust, flax wilt and pasmo.

The final 'Linola' cultivars may thus be the result of selection within the genetically diverse breeding populations generated by intercrossing the aforementioned genotypes. This involves the growing of large populations of plants derived from such crosses and the determination of the status of individual plants for each of the characteristics under consideration. Linolenic acid content can be determined by gas chromatography on oil extracted from the seeds. Yellow seed colour is identified by visual inspection and is characterised descriptively according to classifications in UDSA Technical Bulletin No. 1064, (1953). Yellow seed colour typically falls within a range of yellow described by the Royal Horticultural Society Standard Colour Chart as Yellow 2A-24D (inclusive), Yellow-White 158A-158D (inclusive), Orange-White 159A-159C (inclusive), Greyed Yellow 160A-162D (inclusive), Greyed Orange 163A-163D (inclusive), 164C, 164D, 165C, 165D and 168D.

Seed yield can be determined by weighing the total seed harvested from each plant; disease resistance is evaluated by the severity of symptoms resulting from either natural infection or artificial inoculation by causal organisms; adaptability is determined by relative seed yield when grown over a wide range of diverse environments.

Individual plants, or families of related plants, having some or all of the desired characteristics, or partial combinations thereof, are selected and if necessary intercrossed to combine all characteristics into a single line. These lines are then self-pollinated through several generations until completely inbred. The final selections are true-breeding and are generally uniform in appearance, performance and quality.

Yellow seed coat colour is a preferred characteristic for flax having a low linolenic acid content. The yellow seed coat colour results from either a lack of the usual brown pigmentation in a layer of cells in the seed coat or the complete absence of the pigment-containing cells. In the present invention, this trait is used as an identifying marker to distinguish edible quality low-linolenic flax ('Linola') cultivars from industrial quality high-linolenic cultivars, the overwhelming majority of which are brown-seeded.

The World Flax Collection contains several different lines with the genes required to confer yellow seed colour sufficient for such the purpose of discrimination. All such lines have normal high linolenic acid content and need to be hybridised to the low-linolenic genotypes of the present invention to derive the desired linola genotypes.

Any yellow-seeded line may be regarded as a suitable donor parent to contribute the yellow seeded colour genes to hybrid populations, provided that the other characteristics of the line are such that the resulting hybrid populations are genetically capable of yielding high-performance linola cultivars, and appropriate breeding techniques are subsequently used to so do.

In the present invention, yellow-seed genes have been derived from either of two Commonwealth Plant Introductions, CPI 84495 or CPI 90432. The yellow seed colour of CPI 84495 is conditioned by the action of a single dominant gene, whereas that of CPI 90432 results from the joint action of two recessive genes.

In general, desired 'Linola' genotypes may be obtained by crossing the 'Zero' parent, or lines containing the low-linolenic acid mutations derived from 'Zero', with a yellow-seeded parent, being either CPI 84495, CPI 90432, or breeding lines carrying the yellow-seeded genes derived from either of these two introductions.

Preferred features and characteristics of the second aspect of the invention are as for the first mutatis mutandis.

A third aspect of the present invention relates to a seed obtained from a plant of the first or second aspect. This will generally be flax seed, and preferably has a yellow coat. Other preferred features and characteristics are as for the first and second aspects, mutatis mutandis.

A fourth aspect of the present invention relates to oil derived from seed of the third aspect. This oil is preferably low linolenic acid oil derived from flax seed wherein all carbon-carbon double bonds of the linoleic, linolenic and oleic acids are of the cis geometrical isomer. Suitably the oil has a linolenic acid content of less than about 45% of the total fatty acid content and/or a linoleic acid content in excess of about 10% of the total fatty acid content. It is preferred that this oil comprises a linolenic acid content of from 0% to 30% of total fatty acid content (such as so that the linolenic acid content of the oil is less than 30%). Suitably the linolenic acid content of the oil is less than 30%. Suitably the linoleic acid content of the oil is greater than the linolenic acid content. Preferably the linolenic acid content is less than 15% of the total fatty acid content and more preferably the linolenic acid content is less than 5% of the total fatty acid content. Preferred features and characteristics are as for the previous aspects mutatis mutandis.

A fifth aspect of the present invention relates to a method for producing (flax) seed (that has low linolenic acid content) the method comprising:
(a) inducing a mutation in flax seed; and
(b) selecting flax seed having a seed oil having a linolenic acid content of less than about 45% of the total fatty acid content and optionally a linoleic acid content in excess of about 10% of the total fatty acid content.
More particularly this method comprises:
(a) inducing a mutation in flax seed;
(b) selecting flax seed having a linolenic acid content of less than about 45% of the total fatty acid content and optionally a linoleic acid content in excess of about 10% of the total fatty acid content; and
(c) breeding plants grown from mutant flax seed of (b).
It is preferred that the method involves the chemical induction of mutations. A preferred method comprises:
(a) inducing a mutation in flax seed; and
(b) selecting flax seed having a seed oil having a linolenic acid content of less than about 45% of the total fatty acid content and optionally a linoleic acid content in excess of about 10% of the total fatty acid content.
(c) cross-breeding plants grown from mutant flax seed having low linolenic acid content;
(d) selecting seed from the cross-breeds from (c), having low linolenic acid content;
(e) inbreeding plants grown from the seed selected in (d) and
(f) selecting seed from the inbred plants.
The invention thus encompasses a method of producing flax seed, the method comprising treating flax seed with a chemical (such as a mutagen) or an ionising radiation source to affect the biosynthetic pathway for converting linoleic acid to linolenic acid.

Thus according to one method of the present invention, one or more genetic mutations may be induced in normal flax seeds having a linolenic acid content of from 45% to 65% of total fatty acid content. Methods for inducing genetic mutations are well known. For this invention, the preferred means of inducing mutagenesis is chemical mutagenesis. A number of chemicals are in widespread use as mutagens, including diethyl sulphonate, ethyl methane sulphonate, or the like. The preferred chemical is ethyl methane sulphonate (EMS). Mutagenesis is directed at inducing genetic mutations specifically affecting the linoleic acid desaturation step in fatty acid biosynthesis.

Preferred features and characteristics of the fifth aspect are as for the previous aspects mutatis mutandis.

A sixth aspect of the present invention relates to a method of producing a flax plant, the method comprising growing a plant from seed of the fifth aspect. The method preferably includes producing progeny (such as by cross-breeding) of a flax plant and a plant of the first or second aspect. The method may also include producing the progeny of said flax plant with another flax plant. It is preferred that after any cross-breeding step the plants are inbred. Also preferred is producing progeny by cross-breeding a plant of the present invention with a flax plant having a yellow coated seed. Other preferred features and characteristics are as for previous aspects mutatis mutandis.

The present invention may provide flax seed oil containing all cis-unsaturated carbon-carbon double bonds in the oleic, linoleic and linolenic acids. Such fatty acids may be compared to artificially hydrogenated oils which typically contain mixtures of cis and trans isomers.

The invention can be illustrated by way of Example with reference to the accompanying drawings, in which:

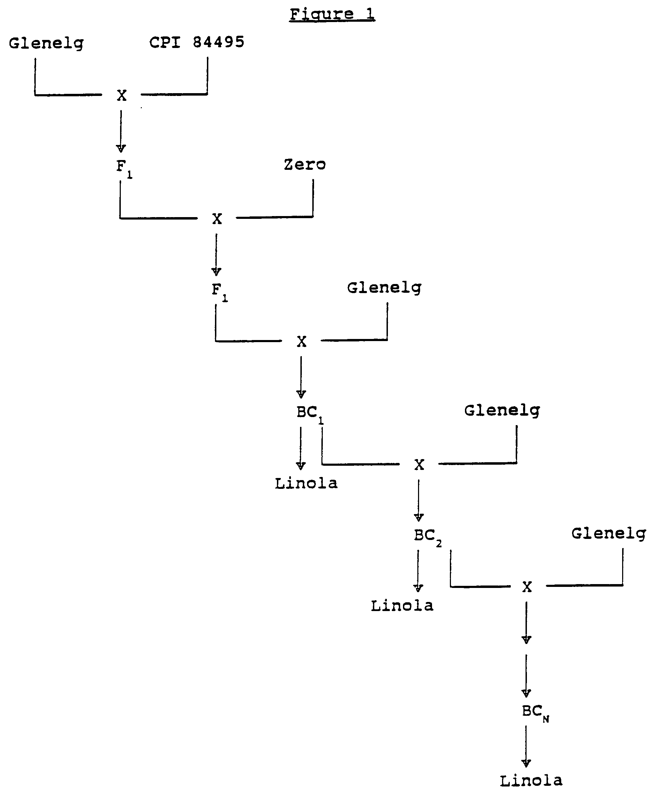

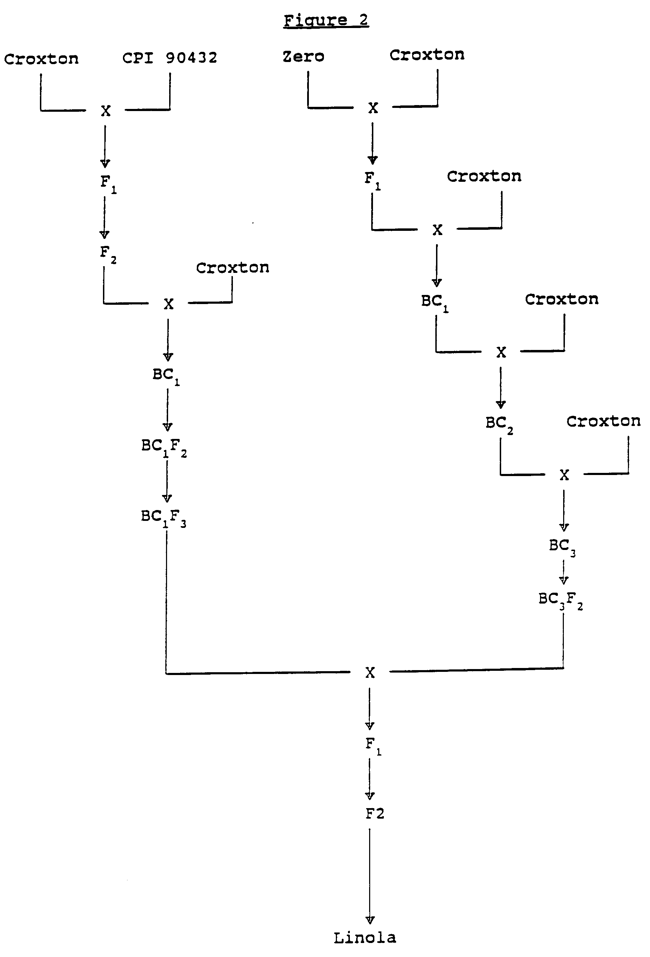

Figures 1 and 2 are diagrams of the production of flax cultivars according to the present invention.

The invention will now be described with reference to the following Examples which are offered by way of illustration and are not to be construed as being limiting.

### EXAMPLE I

### Chemical Mutagenesis

Seeds of Linum usitatissimum cv. Glenelg were restrained in a gauze bundle and soaked in distilled water for 4 hours at 0°C. The soaked seeds were then treated with 0.3% or 0.4% concentrations of the mutagen ethyl methane-sulfonate (EMS) in pH 7.0 phosphate buffer for 2 hours at 2°C, followed by an additional 2 hours at 20°C. Treated seeds were rinsed continuously for 16 hours with cold tap water to inhibit germination and overexposure to the mutagen and dried at room temperature by spreading the seeds over nylon gauze.

Immediately following mutagenic treatment, M₁ seeds were germinated in soil contained in paper tubes and subsequently transplanted to the field in widely spaced rows when approximately three weeks old. At maturity a total of 7072 M₁ plants were harvested from which were obtained 66,500 seeds for analysis. Because fatty acid composition in linseed is determined by the embryo genotype (Yermanos and Knowles, Crop Sci. (1962) 2: 109-11), mutants are selected by analysis of individual M₂ seeds. It is preferable to grow the seedlings at daytime temperatures of 27°C or greater. Warm temperature provides a general downward shift in the mean linolenic acid content and will push the lower extreme of the population into the range where the TBA assay (described in Example II) is most effective.

### COMPARATIVE EXAMPLE II

### Linolenic Acid Content Assay

Up to ten M₂ seeds from each M₁ plant were assayed non-destructively for linolenic acid content in the following manner. Approximately one-quarter of the seed distal to the embryo was removed with a scalpel and the resulting seed "chips" arranged in a grid pattern on an adhesive acetate sheet to which a similar sized Whatman No. 1 chromatography paper was then affixed. A second piece of chromatography paper was then placed on top of the first and the "sandwich" was passed through a pair of metal rollers in order to crush the seed chips and extrude the oil into the free piece of chromatography paper. The resulting test sheets, each containing oil spots from 96 seeds, were left overnight to allow even penetration of the oil into the paper fibers.

The test sheets were analyzed the following day by a modification of the TBA method, a rapid test specific for the oxidation products of linolenic acid in vegetable oils, according to McGregor, Can. J. Plant Sci. (1974) 54:211-13. Specifically, test sheets were exposed to UV radiation for 20 minutes in order to oxidize the linolenic acid, dipped in a 2% solution of thiobarbituric acid (TBA) and then lightly blotted. The TBA solution was freshly prepared by adding 1.8g 2-thiobarbituric acid (4,6-dihydroxy-2-thiopyrimidine) to 60mls of 50% v/v ethanol, heating to completely dissolve the TBA and subsequently adding 30 ml of 20% w/v trichloroacetic acid. Colour development was achieved by placing the individual test sheets between two glass plates clamped together and placed in an oven at 110°C for 20 mins. Mottling of the colour was prevented by placing additional sheets of paper between the glass plates and the test sheet.

The spot colours were compared to those of a standard series of oils varying in linolenic acid concentration, made by combining safflower oil (zero linolenic acid) and linseed oil (45% linolenic acid) in various proportions. Selections were made for low linolenic acid amongst the M₂ seeds and selected plants grown by germinating the untested portion of the seed.

M₂, M₃ and M₄ progeny of the selections were grown under similar glasshouse conditions. Plants of cv. Glenelg that had not been mutagenically treated were grown as controls with each generation. Fatty acid composition was determined by standard gas chromatography procedures according to Green and Marshall, Aust. J. Agric. Res. (1981) 32:599-607 on samples of 30 seeds. Fatty acid composition of cv. Glenelg and homozygous mutant M₃ plants of M1589 and M1722, deposited with the American Type Culture Collection (A.T.C.C.), Rockville, MD, on August 13, 1986 and given A.T.C.C. Accession Nos. 40250 and 40249, respectively, are listed in Table 1.

**Table 1**

| Fatty acid composition of Glenelg and homozygous mutant M₃ plants of M1589 and M1722. | | | | | |
|---|---|---|---|---|---|
| Line | Palmitic (%) | Stearic (%) | Oleic (%) | Linoleic (%) | Linolenic (%) |
| Glenelg | 7.3 | 4.6 | 37.0 | 14.3 | 36.7 |
| M1589 | 7.4 | 5.3 | 42.5 | 24.6 | 20.1 |
| M1722 | 8.2 | 4.7 | 41.5 | 24.4 | 21.0 |

### COMPARATIVE EXAMPLE III

### Determination of Mutation Character

In order to determine if M1589 and M1722 were mutations in the same or different genes, 114 F₂ plants from the cross M1722 x M1589 were analyzed. Linolenic acid varied greatly with five distinct classes apparent, having linolenic acid content means of 1.6%, 15%, 24.0%, 30.5% and 34.5%. The frequency of F₂ plants in these classes were 7, 23, 43, 33 and 8, respectively. These data agree with the genetic ratio of 1:4:6:4:1 (X₄² = 1.89) expected for two unlinked loci exhibiting additive gene action. The seven plants having less than 3% linolenic acid were shown to be homozygous for the mutant alleles at both loci, therefore true breeding, by the occurrence of only plants having less than 3% linolenic acid in their F₃ progeny. One of the plants homozygous for both mutant alleles was designated 'Zero'. Seed derived from 'Zero' were deposited with the ATCC in accordance with the Budapest treaty on August 13, 1986 (Accession No. 40251).

Table 2 provides a fatty acid profile for Glenelg, M1589, M1722 and Zero. The plants were grown under cool conditions (18°C day/13°C night). As indicated in Table 2, the further reduction in linolenic acid content in the 'Zero' line is accompanied by an equivalent increase in the linoleic acid content, indicating that the M1589 and M1722 mutations specifically affect the linoleic desaturation step in fatty acid biosynthesis.

**Table 2**

| Fatty acid composition of Glenelg, M1589, M1722 and 'Zero' grown under an 18°C day/13°C night temperature regime. | | | | | |
|---|---|---|---|---|---|
| Line | Palmitic (%) | Stearic (%) | Oleic (%) | Linoleic (%) | Linolenic (%) |
| Glenelg | 6.0 | 3.4 | 26.3 | 18.8 | 45.4 |
| M1589 | 6.4 | 3.5 | 27.7 | 34.4 | 28.2 |
| M1722 | 6.2 | 3.9 | 31.5 | 30.0 | 28.3 |
| Zero | 6.4 | 3.3 | 25.1 | 62.7 | 2.3 |

### COMPARATIVE EXAMPLE IV

### Effect of Temperature on Production of Linolenic Acid

Cool temperatures such as less than 20°C (day) and 15°C (night), during the period from the date of flowering to date of maturity will tend to increase the proportion of linolenic acid produced by wild-type flax plants. Increased linolenic acid production has also been observed under cool conditions for the mutants described herein. A range of linoleic acid (18:2) and linolenic acid (18:3) is provided in table 3.

**Table 3**

| Variations in Unsaturated 18-Carbon Fatty Acid Compositions in Wild Type and Mutant Flax Seeds | | |
|---|---|---|
| Genotype | Linoleic | Linolenic |
| Glenelg | 15 - 22% | 34 - 50% |
| Glenelg X M1589 and Glenelg X M1722 | 19 - 29% | 27 - 42% |
| M1589 and M1722 | 24 - 35% | 17 - 34% |
| M1589 X Zero and M1722 X Zero | 35 - 52% | 9 - 17% |
| Zero | 46 - 70% | 0 - 4% |

Values are ranges in percentage of total fatty acids encountered over temperature conditions ranging from 15°C/10°C (day/night) to 30°C/25°C (day/night).

### EXAMPLE V

### Yellow coated-Low Linolenic Acid Containing Flax Seed

(A) In this example, plants having similar agronomic characteristics to the flax cultivar Glenelg, but possessing the linola seed quality traits, were developed by transferring the linola genes into the Glenelg genetic background using standard backcross breeding techniques.
   Specifically, the low-linolenic genotype 'Zero' gas crossed to the F₁ of a cross between the flax cultivar Glenelg and CPI 84495, such F₁ being heterozygous for the dominant gene conferring yellow seed colour.
   In the resulting hybrid, plants heterozygous for the dominant yellow-seedcolour gene, and hence having yellow seeds, were backcrossed to plants of the cultivar Glenelg to generate the Backcross 1 population of seeds. Backcross 1 seeds which were genetically heterozygous for both low-linolenic acid genes were identified non-destructively, using standard gas-chromatographic techniques, and grown on for further backcrossing to Glenelg to produce the Backcross 2 seed population.
   By continuing this process through several cycles, populations being heterozygous for the yellow-seed color gene and both of the low-linolenic genes, and showing increasing similarity to the recurrent parent Glenelg, were developed.
   Following each cycle of backcrossing, individuals homozygous for both of the low-linolenic genes and having yellow seeds were selected from the F₂ generations and used to derive fully inbred 'Linola' cultivars by the standard pedigree breeding processes of continued self-pollination and single plant selection. Figure 1 provides an outline of the above selective breeding strategy.
(B) In an alternative embodiment of the present invention, the two recessive yellow-seed genes from CPI 90432 were used in the development of plants having similar agronomic characteristics to the flax cultivar Croxton but possessing the linola seed quality traits.
   In this case, the low-linolenic genes were backcrossed for three cycles into the cultivar Croxton, using similar techniques to those outlined above, to develop a low-linolenic brown-seeded Croxton-type line.
   In parallel, a high-linolenic yellow-seed Croxton-type line was developed by backcrossing the recessive yellow-seed genes into Croxton. Lines homozygous for both the yellow-seed genes were isolated following each backcross cycle by screening F₂ populations for yellow-seeded plants and subsequently crossing these plants to croxton to initiate the next backcross cycle.
   Linola-quality lines were then produced by crossing the low-linolenic brown-seeded Croxton line with the high-linolenic yellow-seeded Croxton line and selecting individual F₂ plants that combined the low-linolenic and yellow-seed traits. Inbred Croxton-type linola cultivars were subsequently developed by the standard pedigree breeding processes of continued self-pollination and single plant selection. Figure 2 provides an outline of the above selective breeding strategy.
   Other 'Linola' cultivars may be readily developed by intercrossing 'Linola' genotypes, such as those produced by the above procedures, or by crossing such 'Linola' genotypes to standard flax genotypes followed by selection of linola-quality progeny and derivation of high-performance inbred lines and other acceptable cultivar structures (e.g., F₁, hybrids, mixtures, or synthetic populations) using a range of standard breeding techniques suitable for self-pollinated crops.

## Claims (Claims for the following Contracting State(s): AT, BE, DE, DK, IT, FR, GR, LU, NL, SE, CH, LI, GB)

1. A flax plant having a genetic mutation, induced by treatment with a chemical or exposure to ionising radiation or by any other non-natural process, which affects the biosynthetic pathway for converting linoleic acid to linolenic acid so that the plant produces a seed oil having a linolenic acid content of less than about 45% of the total fatty acid content.

2. A flax plant according to claim 1 wherein the seed oil further comprises a linoleic acid content in excess of 10% of the total fatty acid content.

3. A flax plant according to claim 1 or 2 wherein the seed oil has a linolenic acid content of about 0% to 30% of total fatty acid content.

4. A flax plant according to any of claims 1 to 3 wherein the seed oil has a linoleic acid content that is greater than the linolenic acid content.

5. A flax plant according to any of claims 1 to 4 having a yellow seed coat.

6. A flax plant which is the progeny of a plant according to any of claims 1 to 5 and having the genetic mutation and producing the seed oil as defined in claim 1.

7. Flax seed derived from a plant according to any of claims 1 to 6 and having the genetic mutation and producing the seed oil as defined in claim 1.

8. Oil derived from flax seed according to claim 7.

9. An oil derived from flax seed wherein all carbon-carbon double bonds of the linoleic, linolenic and oleic acids are of the cis geometrical isomer and having a linolenic acid content of less than about 45% of the total fatty acid content and a linoleic acid content in excess of about 10% of the total fatty acid content.

10. An oil according to claim 9 wherein the oil has a linolenic acid content of from 0% to 30% of total fatty acid content.

11. An oil according to claim 9 or 10 wherein the linoleic acid content of the oil is greater than the linolenic acid content.

12. A method of producing flax seed the method comprising:
(a) inducing a mutation which affects the biosynthetic pathway for converting linoleic acid to linolenic acid in flax seed by treatment with a chemical or exposure to ionising radiation or by any other non-natural process; and
(b) selecting flax seed having a seed oil having a linolenic acid content of less than about 45% of the total fatty acid content and optionally a linoleic acid content in excess of about 10% of the total fatty acid content.

13. A method of producing flax plants, the method comprising:
(a) inducing a mutation which affects the biosynthetic pathway for converting linoleic acid to linolenic acid in flax seed by treatment with a chemical or exposure to ionizing radiation or by any other non-natural process;
(b) selecting flax seed having a linolenic acid content of less than about 45% of the total fatty acid content and optionally a linoleic acid content in excess of about 10% of the total fatty acid content; and
(c) breeding plants grown from mutant flax seed of (b).

14. A method according to claim 13 additionally comprising:
(d) cross-breeding plants in (c) grown from mutant flax seed;
(e) selecting seed from the cross-breeds of (d);
(f) inbreeding plants grown from the seed selected in (e); and
(g) selecting seed from the inbred plants.

15. A method according to claim 14 further comprising:
crossing the plants of (e) with yellow coated seed producing flax and selecting yellow coated seed having low linolenic acid content.

16. Flax seed in which a genetic mutation has been induced by treatment with a chemical or exposure to ionizing radiation or by any other non-natural process which affects the biosynthetic pathway for converting linoleic acid to linolenic acid so that the seed oil has a linolenic acid content of less than about 45% of the total fatty acid content.

## Claims (Claims for the following Contracting State(s): ES)

1. A method of producing a flax plant, the method comprising growing a plant from flax seed having a genetic mutation induced by treatment with a chemical or exposure to ionizing radiation or by any other non-natural process which affects the biosynthetic pathway for converting linoleic acid to linolenic acid so that the plant produces a seed oil having a linolenic acid content of less than about 45% of the total fatty acid content.

2. A method according to claim 1 wherein the seed oil further comprises a linoleic acid content in excess of 10% of the total fatty acid content.

3. A method according to claim 1 or 2 wherein the seed oil has a linolenic acid content of about 0% to 30% of total fatty acid content.

4. A method according to any of claims 1 to 3 wherein the seed oil has a linoleic acid content that is greater than the linolenic acid content.

5. A method according to any of claims 1 to 4 wherein the plant has a yellow seed coat.

6. A method of producing a flax plant, the process comprising crossing a plant produced by the method according to any of claims 1 to 5 with a flax plant, whether produced by such a method or not, the resulting plant having the genetic mutation and producing the seed oil as defined in claim 1.

7. A process for the preparation of flax seed the process comprising obtaining seed from a plant produced by the method according to any of claims 1 to 6, the seed having the genetic mutation and producing the seed oil as defined in claim 1.

8. A process for the production of seed oil, the process comprising extracting oil from flax seed as produced according to claim 7.

9. A process for the production of seed oil the process comprising extracting oil from flax seed wherein all carbon-carbon double bonds of the linoleic, linolenic and oleic acids are of the cis geometrical isomer and having a linolenic acid content of less than about 45% of the total fatty acid content and a linoleic acid content in excess of about 10% of the total fatty acid content.

10. A process according to claim 9 wherein the oil has a linolenic acid content of from 0% to 30% of total fatty acid content.

11. A process for the production of an oil according to claim 9 or 10 wherein the linoleic acid content of the oil is greater than the linolenic acid content.

12. A method of producing flax seed the method comprising:
(a) inducing a mutation which affects the biosynthetic pathway for converting linoleic acid to linolenic acid in flax seed by treatment with a chemical or exposure to ionizing radiation or by any other non-natural process; and
(b) selecting flax seed having a seed oil having a linolenic acid content of less than about 45% of the total fatty acid content and optionally a linoleic acid content in excess of about 10% of the total fatty acid content.

13. A method of producing flax plants, the method comprising:
(a) inducing a mutation which affects the biosynthetic pathway for converting linoleic acid to linolenic acid in flax seed by treatment with a chemical or exposure to ionizing radiation or by any other non-natural process;
(b) selecting flax seed having a linolenic acid content of less than about 45% of the total fatty acid content and optionally a linoleic acid content in excess of about 10% of the total fatty acid content; and
(c) breeding plants grown from mutant flax seed of (b).

14. A method according to claim 13 additionally comprising:
(d) cross-breeding plants in (c) grown from mutant flax seed;
(e) selecting seed from the cross-breeds of (d);
(f) inbreeding plants grown from the seed selected in (e); and
(g) selecting seed from the inbred plants.

15. A method according to claim 14 further comprising:
crossing the plants of (e) with yellow coated seed producing flax and selecting yellow coated seed having low linolenic acid content.

## Patentansprüche (Patentansprüche für folgende(n) Vertragsstaat(en): AT, BE, DE, DK, IT, FR, GR, LU, NL, SE, CH, LI, GB)

1. Flachspflanze, die eine genetische Mutation trägt, die durch Behandlung mit einer Chemikalie oder Exposition gegenüber einer ionisierenden Strahlung oder durch irgendein anderes, nicht natürliches verfahren induziert wurde, und die den Biosynthese-Weg der Umwandlung von Linolsäure zu Linolensäure beeinflußt, so daß die Pflanze ein Samenöl herstellt, das einen Linolensäure-Gehalt von weniger als etwa 45% des Gesamt-Fettsäure-Gehalts hat.

2. Flachspflanze nach Anspruch 1, deren Samenöl ferner einen Linolsäure-Gehalt von mehr als 10% des Gesamt-Fettsäure-Gehalts hat.

3. Flachspflanze nach den Ansprüchen 1 oder 2, deren Samenöl ein Linolensäure-Gehalt von etwa 0 bis 30% des Gesamt-Fettsäure-Gehalts hat.

4. Flachspflanze nach einem der Ansprüche 1 bis 3, deren Samenöl einen Linolsäure-Gehalt hat, der größer als der Linolensäure-Gehalt ist.

5. Flachspflanze nach einem der Ansprüche 1 bis 4 mit einer gelben Samenschale.

6. Flachspflanze, die ein Nachkomme einer Pflanze nach einem der Ansprüche 1 bis 5 ist, und die eine genetische Mutation trägt und Samenöl herstellt, wie in Anspruch 1 definiert.

7. Flachssamen, der von einer Pflanze nach einem der Ansprüche 1 bis 6 stammt, und die genetische Mutation trägt und Samenöl herstellt, wie in Anspruch 1 definiert.

8. Öl, das aus Flachssamen nach Anspruch 7 gewonnen wurde.

9. Öl, das aus Flachssamen gewonnen wurde, dessen KohlenstoffKohlenstoff-Doppelbindungen der Linol-, Linolen- und Ölsäure alle in der geometrischen cis-Isomer-Form vorliegen, und das einen Linolensäure-Gehalt von weniger als etwa 45% des Gesamt-Fettsäure-Gehalts aufweist und einen Linolsäure-Gehalt von mehr als etwa 10% des Gesamt-Fettsäure-Gehalts.

10. Öl nach Anspruch 9, wobei das Öl einen Linolensäure-Gehalt von 0% bis 30% des Gesamt-Fettsäure-Gehalts hat.

11. Öl nach den Ansprüchen 9 oder 10, wobei der Linolsäure-Gehalt des Öls größer als der Linolensäure-Gehalt ist.

12. Verfahren zur Herstellung von Flachssamen, dadurch gekennzeichnet, daß man:
(a) eine Mutation induziert, die den Biosyntheseweg der Umwandlung von Linolsäure zu Linolensäure in Flachssamen durch Behandlung mit einer Chemikalie oder Exposition gegenüber einer ionisierenden Strahlung oder durch irgendein anderes, nicht natürliches verfahren beeinflußt; und
(b) Flachssamen auswählt, der Samenöl mit einem Linolensäure-Gehalt von weniger als etwa 45% des Gesamt-Fettsäure-Gehalts und gegebenenfalls einen Linolsäure-Gehalt von mehr als etwa 10% des Gesamt-Fettsäure-Gehalts hat.

13. Verfahren zur Herstellung von Flachspflanzen, dadurch gekennzeichnet, daß man
(a) eine Mutation induziert, die den Biosyntheseweg der Umwandlung von Linolsäure zu Linolensäure in Flachssamen durch Behandlung mit einer Chemikalie oder Exposition gegenüber einer ionisierenden Strahlung oder durch irgendein anderes, nicht natürliches Verfahren beeinträchtigt;
(b) Flachssamen auswählt, der einen Linolensäure-Gehalt von weniger als etwa 45% des Gesamt-Fettsäure-Gehalts und gegebenenfalls einen Linolsäure-Gehalt von mehr als etwa 10% des Gesamt-Fettsäure-Gehalts hat; und
(c) Pflanzen züchtet, die aus dem mutierten Flachssamen von (b) gewachsen sind.

14. Verfahren nach Anspruch 13, bei dem man zusätzlich:
(d) Pflanzen kreuzt, die aus mutiertem Flachssamen in (c) gewachsen sind;
(e) Samen aus den Kreuzungen von (d) auswählt;
(f) Inzucht mit den Pflanzen betreibt, die aus dem Samen gewachsen sind, der in (e) ausgewählt wurde; und
(g) Samen aus den Inzucht-Pflanzen auswählt.

15. Verfahren nach Anspruch 14, bei dem man darüber hinaus:
die Pflanzen aus (e) mit einem Flachs kreuzt, der gelbe Samenschalen hat, und Samen mit gelben Schalen und mit geringem Linolensäure-Gehalt auswählt.

16. Flachssamen, bei dem eine genetische Mutation durch Behandlung mit einer Chemikalie oder Exposition gegenüber einer ionisierenden Strahlung oder durch irgendein anderes, nicht natürliches Verfahren induziert wurde, die den Biosyntheseweg der Umwandlung von Linolsäure zu Linolensäure so beeinflußt, daß das Samenöl einen Linolensäure-Gehalt von weniger als etwa 45% des Gesamt-Fettsäure-Gehalts enthält.

## Patentansprüche (Patentansprüche für folgende(n) Vertragsstaat(en): ES)

1. Verfahren zur Herstellung einer Flachspflanze, dadurch gekennzeichnet, daß man eine Pflanze aus Flachssamen zieht, der eine genetische Mutation trägt, die durch Behandlung mit einer Chemikalie oder Exposition gegenüber einer ionisierenden Strahlung oder durch irgendein anderes, nicht natürliches Verfahren induziert wurde, und die den Biosynthese-Weg der Umwandlung von Linolsäure zu Linolensäure beeinflußt, so daß die Pflanze ein Samenöl herstellt, das einen Linolensäure-Gehalt von weniger als etwa 45% des Gesamt-Fettsäure-Gehalts hat.

2. Verfahren nach Anspruch 1, bei dem das Samenöl ferner einen Linolsäure-Gehalt von mehr als 10% des Gesamt-Fettsäure-Gehalts hat.

3. Verfahren nach den Ansprüchen 1 oder 2, bei dem das Samenöl ein Linolensäure-Gehalt von etwa 0 bis 30% des Gesamt-Fettsäure-Gehalts hat.

4. Verfahren nach einem der Ansprüche 1 bis 3, bei dem das Samenöl einen Linolsäure-Gehalt hat, der größer als der Linolensäure-Gehalt ist.

5. Verfahren nach einem der Ansprüche 1 bis 4, wobei die Pflanze eine gelbe Samenschale hat.

6. Verfahren zur Herstellung einer Flachspflanze, dadurch gekennzeichnet, daß man eine Pflanze, die nach einem der in den Ansprüchen 1 bis 5 dargestellten Verfahren hergestellt wurde, mit einer Flachspflanze kreutzt, die nach einem derartigen Verfahren hergestellt wurde oder nicht, wobei die resultierende Pflanze eine genetische Mutation trägt und Samenöl herstellt, wie in Anspruch 1 definiert.

7. Verfahren zur Herstellung von Flachssamen, dadurch gekennzeichnet, daß man Samen gewinnt, der von einer Pflanze stammt, die nach einem der Ansprüche 1 bis 6 hergestellt wurde, und der Samen die genetische Mutation trägt und Samenöl herstellt, wie in Anspruch 1 definiert.

8. Verfahren zur Herstellung von Samenöl, dadurch gekennzeichnet, daß man Öl aus Flachssamen extrahiert, der nach Anspruch 7 hergestellt wurde.

9. Verfahren zur Herstellung von Samenöl, dadurch gekennzeichnet, daß man Öl aus Flachssamen extrahiert, dessen Kohlenstoff-Kohlenstoff-Doppelbindungen der Linol-, Linolen- und Ölsäure alle in der geometrischen cis-Isomer-Form vorliegen, und das einen Linolensäure-Gehalt von weniger als etwa 45% des Gesamt-Fettsäure-Gehalts aufweist und einen Linolsäure-Gehalt von mehr als etwa 10% des Gesamt-Fettsäure-Gehalts.

10. Verfahren nach Anspruch 9, wobei das Öl einen Linolensäure-Gehalt von 0% bis 30% des Gesamt-Fettsäure-Gehalts hat.

11. Verfahren zur Herstellung von Öl nach den Ansprüchen 9 oder 10, bei dem der Linolsäure-Gehalt des Öls größer als der Linolensäure-Gehalt ist.

12. Verfahren zur Herstellung von Flachssamen, dadurch gekennzeichnet, daß man:
(a) eine Mutation induziert, die den Biosyntheseweg der Umwandlung von Linolsäure zu Linolensäure in Flachssamen durch Behandlung mit einer Chemikalie oder Exposition gegenüber einer ionisierenden Strahlung oder durch irgendein anderes, nicht natürliches Verfahren beeinflußt; und
(b) Flachssamen auswählt, der Samenöl mit einem Linolensäure-Gehalt von weniger als etwa 45% des Gesamt-Fettsäure-Gehalts und gegebenenfalls einen Linolsäure-Gehalt von mehr als etwa 10% des Gesamt-Fettsäure-Gehalts hat.

13. Verfahren zur Herstellung von Flachspflanzen, dadurch gekennzeichnet, daß man
(a) eine Mutation induziert, die den Biosyntheseweg der Umwandlung von Linolsäure zu Linolensäure in Flachssamen durch Behandlung mit einer Chemikalie oder Exposition gegenüber einer ionisierenden Strahlung oder durch irgendein anderes, nicht natürliches Verfahren beeinträchtigt;
(b) Flachssamen auswählt, der einen Linolensäure-Gehalt von weniger als etwa 45% des Gesamt-Fettsäure-Gehalts und gegebenenfalls einen Linolsäure-Gehalt von mehr als etwa 10% des Gesamt-Fettsäure-Gehalts hat; und
(c) Pflanzen züchtet, die aus dem mutierten Flachssamen von (b) gewachsen sind.

14. Verfahren nach Anspruch 13, bei dem man zusätzlich:
(d) Pflanzen kreuzt, die aus mutiertem Flachssamen in (c) gewachsen sind;
(e) Samen aus den Kreuzungen von (d) auswählt;
(f) Inzucht mit den Pflanzen betreibt, die aus dem Samen gewachsen sind, der in (e) ausgewählt wurde; und
(g) Samen aus den Inzucht-Pflanzen auswählt.

15. Verfahren nach Anspruch 14, bei dem man darüber hinaus:
die Pflanzen aus (e) mit einem Flachs kreuzt, der gelbe Samenschalen hat, und Samen mit gelben Schalen und mit geringem Linolensäure-Gehalt auswählt.

## Revendications (Revendications pour l'(les) Etat(s) contractant(s) suivant(s): AT, BE, DE, DK, IT, FR, GR, LU, NL, SE, CH, LI, GB)

1. Végetal de type lin oléagineux présentant une mutation génétique induite par traitement avec un produit chimique ou par exposition à un rayonnement ionisant ou par un autre procédé non naturel, laquelle affecte le mode de biosynthèse de transformation de l'acide linoléique en acide linolénique de sorte que le végétal produit une huile de graines ayant une teneur en acide linolénique inférieure à environ 45% de la teneur totale en acides gras.

2. Végétal de type lin oléagineux selon la revendication 1, dans lequel l'huile de haines comprend de plus une teneur en acide linoléique de plus de 10% de la teneur totale en acides gras.

3. Végétal de type lin oléagineux selon la revendication 1 ou 2, dans lequel l'huile de graines possède une teneur en acide linolénique comprise entre environ 0% et 30% de la teneur totale en acides gras.

4. Végétal de type lin oléagineux selon l'une quelconque des revendications 1 à 3, dans lequel l'huile de graines possède une teneur en acide linoléique qui est supérieure à la teneur en acide linolénique.

5. Végétal de type lin oléagineux selon l'une quelconque des revendications 1 à 4, ayant une enveloppe jaune de graine.

6. Végétal de type lin oléagineux qui est le descendant d'un végétal selon l'une quelconque des revendications 1 à 5 et qui a la mutation génétique et produit l'huile de graines telle que définie dans la revendication 1.

7. Semence de lin oléagineux provenant d'un végétal selon l'une quelconque des revendications 1 à 6 et ayant la mutation génétique et produisant l'huile de graines telle que définie dans la revendication 1.

8. Huile provenant de la semence de lin oléagineux selon la revendication 7.

9. Huile provenant de la semence de lin oléagineux dans laquelle toutes les liaisons doubles carbone-carbone des acides linoléiques, linoléniques et oléiques sont du type isomère géométrique cis et ayant une teneur en acide linolénique inférieure à environ 45% de la teneur totale en acides gras et une teneur en acide linoléique de plus d'environ 10% de la teneur totale en acides gras.

10. Huile selon la revendication 9, dans laquelle l'huile possède une teneur en acide linolénique comprise entre 0% et 30% de la teneur totale en acides gras.

11. Huile selon la revendication 9 ou 10, dans laquelle la teneur en acide linoléique de l'huile est supérieure à la teneur en acide linolénique.

12. Procédé de production de semence de lin oléagineux, le procédé comprenant :
(a) l'induction d'une mutation qui affecte le mode de biosynthèse de transformation de l'acide linoléique en acide linolénique dans la semence de lin oléagineux par traitement avec un produit chimique ou par exposition à un rayonnement ionisant ou par un autre procédé non naturel ; et
(b) la sélection de la semence de lin oléagineux présentant une huile de graines ayant une teneur en acide linolénique inférieure à environ 45% de la teneur totale en acides gras et éventuellement une teneur en acide linoléique de plus d'environ 10% de la teneur totale en acides gras.

13. Procédé de production de végétaux de type lin oléagineux, le procédé comprenant :
(a) l'induction d'une mutation qui affecte le mode de biosynthèse de transformation de l'acide linoléique en acide linolénique dans la semence de lin oléagineux par traitement avec un produit chimique ou par exposition à un rayonnement ionisant ou par un autre procédé non naturel ;
(b) la sélection de la semence de lin oléagineux ayant une teneur en acide linolénique inférieure à environ 45% de la teneur totale en acides gras et éventuellement une teneur en acide linoléique de plus d'environ 10% de la teneur totale en acides gras ; et
(c) la reproduction des végétaux issus de la semence de lin oléagineux mutant de (b).

14. Procédé selon la revendication 13 comprenant de plus :
(d) le croisement des végétaux de (c) issus de la semence de lin oléagineux mutant ;
(e) la sélection de la semence parmi les croisements de (d) ;
(f) la sélection consanguine des végétaux issus de la semence sélectionnée dans (e) ; et
(g) la sélection de la semence parmi les végétaux consanguins.

15. Procédé selon la revendication 14 comprenant de plus : le croisement des végétaux de (e) avec du lin oléagineux produisant des graines à enveloppe jaune et la sélection de la semence à enveloppe jaune présentant une teneur faible en acide linolénique.

16. Semence de lin oléagineux dans laquelle une mutation génétique a été induite par traitement avec un produit chimique ou par exposition à un rayonnement ionisant ou par un autre procédé non naturel, laquelle affecte le mode de biosynthèse de transformation de l'acide linoléique en acide linolénique de sorte que l'huile de graines possède une teneur en acide linolénique inférieure à environ 45% de la teneur totale en acides gras.

## Revendications (Revendications pour l'(les) Etat(s) contractant(s) suivant(s): ES)

1. Procédé de production d'un végétal de type lin oléagineux, le procédé comprenant la culture d'un végétal à partir de semence de lin oléagineux présentant une mutation génétique induite par traitement avec un produit chimique ou par exposition à un rayonnement ionisant ou par un autre procédé non naturel, laquelle affecte le mode de biosynthèse de transformation de l'acide linoléique en acide linolénique de sorte que le végétal produit une huile de graines ayant une teneur en acide linolénique inférieure à environ 45% de la teneur totale en acides gras.

2. Procédé selon la revendication 1, dans lequel l'huile de graines comprend de plus une teneur en acide linoléique de plus de 10% de la teneur totale en acides gras.

3. Procédé selon la revendication 1 ou 2, dans lequel l'huile de graines possède une teneur en acide linolénique comprise entre environ 0% et 30% de la teneur totale en acides gras.

4. Procédé selon l'une quelconque des revendications 1 à 3, dans lequel l'huile de graines possède une teneur en acide linoléique qui est supérieure à la teneur en acide linolénique.

5. Procédé selon l'une quelconque des revendications 1 à 4, dans lequel le végétal possède une enveloppe jaune de graine.

6. Procédé de production d'un végétal de type lin oléagineux, le procédé comprenant le croisement d'un végétal produit par le procédé selon l'une quelconque des revendications 1 à 5 avec un végétal de type lin oléagineux produit ou non par un tel procédé, le végétal résultant ayant la mutation génétique et produisant l'huile de graines telle que définie dans la revendication 1.

7. Procédé de préparation de semence de lin oléagineux, le procédé comprenant l'obtention de la semence à partir d'un végétal produit par le procédé selon l'une quelconque des revendications 1 à 6, la semence ayant la mutation génétique et produisant l'huile de graines telle que définie dans la revendication 1.

8. Procédé de production d'huile de graines, le procédé comprenant l'extraction de l'huile à partir de la semence de lin oléagineux telle que produite selon la revendication 7.

9. Procédé de production d'huile de graines, le procédé comprenant l'extraction de l'huile à partir de la semence de lin oléagineux dans laquelle toutes les liaisons doubles carbone-carbone des acides linoléiques, linoléniques et oléiques sont du type isomère géométrique cis et qui a une teneur en acide linolénique inférieure à environ 45% de la teneur totale en acides gras et une teneur en acide linoléique de plus d'environ 10% de la teneur totale en acides gras.

10. Procédé selon la revendication 9, dans lequel l'huile possède une teneur en acide linolénique comprise entre 0% et 30% de la teneur totale en acides gras.

11. Procédé de production d'huile selon la revendication 9 ou 10, dans lequel la teneur en acide linoléique de l'huile est supérieure à la teneur en acide linolénique.

12. Procédé de production de semence de lin oléagineux, le procédé comprenant :
(a) l'induction d'une mutation qui affecte le mode de biosynthèse de transformation de l'acide linoléique en acide linolénique dans la semence de lin oléagineux par traitement avec un produit chimique ou par exposition à un rayonnement ionisant ou par un autre procédé non naturel ; et
(b) la sélection de la semence de lin oléagineux présentant une huile de graines ayant une teneur en acide linolénique inférieure à environ 45% de la teneur totale en acides gras et éventuellement une teneur en acide linoléique de plus d'environ 10% de la teneur totale en acides gras.

13. Procédé de production de végétaux de type lin oléagineux, le procédé comprenant :
(a) l'induction d'une mutation qui affecte le mode de biosynthèse de transformation de l'acide linoléique en acide linolénique dans la semence de lin oléagineux par traitement avec un produit chimique ou par exposition à un rayonnement ionisant ou par un autre procédé non naturel ;
(b) la sélection de la semence de lin oléagineux ayant une teneur en acide linolénique inférieure à environ 45% de la teneur totale en acides gras et éventuellement une teneur en acide linoléique de plus d'environ 10% de la teneur totale en acides gras ; et
(c) la reproduction des végétaux issus de la semence de lin oléagineux mutant de (b).

14. Procédé selon la revendication 13 comprenant de plus :
(d) le croisement des végétaux de (c) issus de la semence de lin oléagineux mutant ;
(e) la sélection de la semence parmi les croisements de (d) ;
(f) la sélection consanguine des végétaux issus de la semence sélectionnée dans (e) ; et
(g) la sélection de la semence parmi les végétaux consanguins.

15. Procédé selon la revendication 14 comprenant de plus : le croisement des végétaux de (e) avec du lin oléagineux produisant des graines à enveloppe jaune et la sélection de la semence à enveloppe jaune présentant une teneur faible en acide linolénique.
